# EUROPÄISCHE PATENTSCHRIFT

(11) **EP 4 376 753 B1**
(45) Veröffentlichungstag und Bekanntmachung des Hinweises auf die Patenterteilung: **09.12.2026**
(21) Anmeldenummer: 22757531.3
(22) Anmeldetag: 25.07.2022
(51) Int. Cl.: A61B 34/30, A61B 34/00, A61B 1/005, A61B 17/00, A61B 17/29

(54) **VERFAHREN ZUM MONTIEREN EINER GELENKMECHANIK FÜR EIN MEDIZINISCHES INSTRUMENT SOWIE GELENKMECHANIK FÜR EIN MEDIZINISCHES INSTRUMENT UND MEDIZINISCHES INSTRUMENT**
METHOD OF ASSEMBLING AN ARTICULATION MECHANISM FOR A MEDICAL INSTRUMENT AND AN ARTICULATION MECHANISM FOR A MEDICAL INSTRUMENT AND MEDICAL INSTRUMENT
PROCEDE D'ASSEMBLAGE D'UN MECANISME D'ARTICULATION POUR INSTRUMENT MEDICAL ET MECANISME D'ARTICULATION POUR INSTRUMENT MEDICAL ET INSTRUMENT MEDICAL

(30) Priorität: 28.07.2021 DE 102021119513
(43) Veröffentlichungstag der Anmeldung: 05.06.2024
(73) Patentinhaber: Karl Storz SE & Co. KG, 78532 Tuttlingen (DE)
(72) Erfinder: LÄNGLE, Dominik, 78532 Tuttlingen (DE); SCHNEIDER, Janosz, 78532 Tuttlingen (DE); STEFAN, Jochen, 78532 Tuttlingen (DE); ENGESSER, Christian, 78532 Tuttlingen (DE)
(86) Internationale Anmeldenummer: PCT/EP2022/070770
(87) Internationale Veröffentlichungsnummer: WO 2023/006652

(56) Entgegenhaltungen:
- EP-A1- 3 738 540
- EP-A1- 3 772 343
- US-A1- 2018 071 490

## Beschreibung

Die Erfindung betrifft ein Verfahren zum Montieren einer Gelenkmechanik für ein medizinisches Instrument, insbesondere für einen medizinischen Roboter oder für ein Endoskop, wobei die Gelenkmechanik eine stützende Gelenkanordnung mit einem Gelenkkörper oder mehreren Gelenkkörpern, mehrere mit einem distalen Endabschnitt verbundene, um die Gelenkanordnung angeordnete Lenkelemente zum Manipulieren des distalen Endabschnitts und einer an einem proximalen Abschnitt angeordnete Taumelscheibe zum mechanischen Ansteuern der Lenkelemente aufweist. Weiterhin betrifft die Erfindung eine Gelenkmechanik für ein medizinisches Instrument, insbesondere für einen medizinischen Roboter oder für ein Endoskop, wobei die Gelenkmechanik eine stützende Gelenkanordnung mit einem Gelenkkörper oder mehreren Gelenkkörpern, mehrere mit einem distalen Endabschnitt verbundene, um die Gelenkanordnung angeordnete Lenkelemente zum Manipulieren des distalen Endabschnitts und einer an einem proximalen Abschnitt angeordneten Taumelscheibe zum mechanischen Ansteuern der Lenkelemente aufweist. Weiterhin betrifft die Erfindung ein medizinisches Instrument, insbesondere einen medizinischen Roboter oder ein Endoskop mit einer Gelenkmechanik.

Gelenkmechaniken der genannten Art werden insbesondere als sogenannte distale Gelenkmechaniken für medizinische Instrumente hergestellt. An solchen Gelenkmechaniken angebrachte Lenkelemente, welche oft als Lenkdrähte aufgeführt sind, welche zum Steuern und/oder Ausrichten der Gelenkmechanik genutzt werden, müssen in einem solchen System einheitlich und definiert vorgespannt werden. Dies dient einer genauen oder auch gleichmäßigen Abwinkelung und/oder auch einer Selbstausrichtung des jeweiligen Systems. Für das Vorspannen von Lenkdrähten in einem Roboterendeffektor oder einem Endoskopendeffektor oder einem Endeffektor für ein anders medizinisches Instrument sind bislang beispielsweise Lösungen bekannt, bei welchen jeder einzelne Lenkdraht mit einer Schraubverbindung oder einer anderen Verbindung definiert gespannt wird. Dieser Montageprozess ist sehr zeitintensiv. Insbesondere muss jeder Lenkdraht zuerst mit einem Gewicht auf eine Vorspannung gebracht werden. Anschließend muss eine entsprechende Verbindung und/oder Befestigung des jeweiligen Lenkdrahtes hergestellt werden. Oftmals kommt es bereits bei diesem Schritt zu einer Vorschädigung eines Lenkdrahtes. Zudem ist eine entsprechende Verbindungstechnik zumeist platzintensiv oder auch aufwendig zu realisieren.

EP 3 772 343 B1 beschreibt ein medizinisches Instrument mit einem hohlen Schaft, an dessen distalem Ende des Schaftes ein angeordnetes Instrument mittels eines Gelenkmechanismus relativ zur Längsachse des Schaftes verschwenkbar ist, wobei der Gelenkmechanismus über in Längsrichtung des Schaftes verlaufende Lenkdrähte mit einem proximalen Antrieb verbunden ist. Die Lenkdrähte sind an der räumlich verstellbaren Scheibe des proximalen Antriebs mittels Klemmverbindungen festgelegt, indem in der räumlich verstellbaren Scheibe Durchgangsbohrungen für jeden Lenkdraht ausgebildet sind, wobei die Lenkdrähte innerhalb der Durchgangsbohrungen jeweils über eine Madenschraube fixiert und kraftschlüssig mit der räumlich verstellbaren Scheibe verbunden sind.

Die US 2018/0071490 A1 beschreibt eine steuerbare Katheteranordnung, bei welcher eine Kardanplatte schwenkbar in einem kardanischen Ring angeordnet ist und Zugdrähte einzeln mittels jeweils eines Drahteingriffes an der Kardanplatte befestigt sind. Des Weiteren weist die Katheteranordnung an ihrem proximalen Ende ein Steuergriff mit einem Abwinkelungsknopf und einen Steuerknopf auf, wobei mittels des Abwinkelungsknopfes eine Stärke einer Abwinklung des distalen Endabschnittes des Katheters einstellbar und mittels eines Antreibens des Steuerknopfes und eines mit diesem festverbundenen Vorsprungselementes in distaler Richtung die Kardanplatte schwenkbar ist. Mittels eines Kupplungsknopfes in einer eingerückten Position kann der Steuerknopf derart eingerastet werden, dass ein umlaufender Winkel der distalen Spitze des verbundenen Katheters fixiert ist, während der Abwinkelungsknopf weiterhin das Vorsprungselement axial antreiben und dadurch das Ausmaß der Abwinkelung der distalen Spitze des Katheters verändern kann.

Die US 7,699,855 offenbart eine Schnittstelle, um Instrumente oder andere Endeffektoren an einem Roboterarm anzuschließen. Dabei befinden sich Antriebe für Funktionen des Instrumentes auf der Seite des Roboters, womit das Instrument kostengünstig bleibt. Die Übertragung von Drehwinkeln von Antrieben zum Instrument erfolgt mittels Kupplungsscheiben in einer gemeinsamen Trennebene. Achsen der Antriebe stehen deshalb parallel senkrecht zu dieser Ebene.

Die WO 00/2014004242 beschreibt eine weitere Schnittstelle für sogenannte achsparallele Antriebe für einen Greifmechanismus eines chirurgischen Instrumentes.

In der US 10,105,128 wird eine Mechanik zum Ausrichten einer Taumelscheibe mittels Gelenkstangen mit zwei achsparallelen Antrieben beschrieben.

Die EP 3 738 540 A1 betrifft ein flexibles chirurgisches Instrumentensystem mit einem distalseitigen chirurgischen Endeffektor, welcher durch Drücken und Ziehen eines Betätigungsdrahts geöffnet oder geschlossen werden kann, indem durch Drehen einer Schraube eines Endeffektor-Antriebsmechanismus sich ein Gleitblock auf der Schraube linear entlang der Führungsstange auf- und abbewegt.

Aufgabe der Erfindung ist es, den Stand der Technik zu verbessern.

Gelöst wird diese Aufgabe durch ein Verfahren zum Montieren einer Gelenkmechanik für ein medizinisches Instrument, insbesondere für einen medizinischen Roboter oder für ein Endoskop, wobei die Gelenkmechanik eine stützende Gelenkanordnung mit einem Gelenkkörper oder mehreren Gelenkkörpern, mehrere mit einem distalen Endabschnitt verbundene, um die Gelenkanordnung angeordnete Lenkelement zum Manipulieren des distalen Endabschnitts und eine an einem proximalen Abschnitt angeordnete Taumelscheibe zum mechanischen Ansteuern der Lenkelemente aufweist, mit folgenden Schritten:
- Vormontieren der Gelenkanordnung mit einer Taumelscheibe und dem distalen Endabschnitt sowie den Lenkelementen, sodass eine Gelenkanordnung mit dem distalen Endabschnitt, den Lenkelementen und der Taumelscheibe vorliegt und die Lenkelemente zugfest mit dem distalen Endabschnitt verbunden sind,
- Befestigen der Lenkelemente an der Taumelscheibe, sodass die Lenkelemente zwischen dem distalem Endabschnitt und der Taumelscheibe zugfest und/oder druckfest fixiert sind,
- Spannen der Lenkelemente mittels eines axialen Verschiebens der Taumelscheibe mittels einer Verschiebeeinrichtung in proximaler Richtung, sodass die Lenkelemente mit einer Montagespannung zwischen dem distalen Endbereich und der Taumelscheibe gespannt sind,
- Festsetzen der Taumelscheibe an der Gelenkanordnung in axialer Richtung mittels eines Arretierens der Verschiebeeinrichtung,
sodass die Gelenkmechanik montiert und mit gespannten Lenkelementen vorliegt.

Durch ein solches Verfahren ist es möglich, nach dem Vormontieren der Gelenkanordnung und dem Befestigen der Lenkelemente in einem einzigen Schritt mittels eines Spannens und Festsetzens der Taumelscheibe alle Lenkelemente gleichzeitig und somit gemeinsam zu spannen. Es erfolgt nur ein einziges Festsetzen der Taumelscheibe an der Gelenkanordnung und damit ein einziges Festsetzen aller gespannten Lenkelemente gleichzeitig in einem Schritt. Zudem ist eine solche Gelenkmechanik durch eine einheitliche Spannung aller Lenkelemente möglichst genau ausgerichtet.

Folgende Begriffe seien an dieser Stelle erläutert:
Eine "Gelenkmechanik" kann jede mechanische Anordnung sein, welche dazu geeignet ist, in einer oder mehreren Achsen verschwenkt zu werden und welche dazu dient, einen Endeffektor für eine medizinische Anwendung zu bewegen oder zu schwenken. Beispielsweise ist eine solche Gelenkmechanik mit einer Gelenkanordnung aus einer Abfolge von "Gelenkkörpern" ausgebildet, wobei die Gelenkkörper jeweils aufeinander abwälzen und damit eine vielfältige Gelenkigkeit der Gelenkanordnung und damit der Gelenkmechanik darstellen.

Ein "medizinisches Instrument" kann jede mechanische oder mechanisch-elektrische Wirkeinheit sein, welche dazu geeignet ist, für medizinische Behandlungen eines Menschen oder eines Tieres eingesetzt zu werden, insbesondere ein Endoskop oder auch ein medizinischer Roboter. Beispielsweise kann ein solches medizinisches Instrument ein Schneidwerkzeug, ein Greifwerkzeug, ein optisches System zur Begutachtung eines Sichtbereiches, ein kombiniertes Instrument aus den unterschiedlichen aufgezählten Möglichkeiten oder ein anderes für ein medizinisches Behandlungsverfahren geeignetes Werkzeug oder Instrument aufnehmen. Beispielsweise kann ein solches medizinisches Instrument auch ein separat von einem Endoskop gelieferter Schaft eines Endoskopes sein.

Ein "medizinischer Roboter" ist eine technische Apparatur, welche üblicherweise dazu geeignet ist, wiederkehrende oder definierte mechanische Arbeit zu übernehmen. Roboter sind sowohl als ortsfeste als auch als mobile Maschinen bekannt und werden von entsprechender Software gesteuert. Roboter verfügen dabei über Antriebsmotoren, beispielsweise zum Antreiben unterschiedlicher Bewegungsachsen eines Roboters, sodass entsprechende Kräfte auf einen Endeffektor aufgebracht werden können. Ein solcher Roboter kann im vorliegenden Beispiel aber auch eine sehr kleine, filigran arbeitende technische Einheit sein, welche bei medizinischen Eingriffen oder der medizinischen Behandlung eines Menschen oder eines Tieres unterstützt und beispielsweise ein Endoskop trägt und entsprechend positioniert.

Ein "Endoskop" ist ein technisches und/oder medizinisches Instrument, welches beispielsweise dazu genutzt wird, Hohlräume oder schlecht zugängliche Bereiche optische zu inspizieren und/oder mittels des Endoskopes auch Manipulatoren oder Endeffektoren einzusetzen, um eine bestimmte Tätigkeit in einem Arbeitsraum zu verrichten. Beispielsweise ist ein solches Endoskop mit einem beweglichen und/oder schwenkbaren Schaft an seinem Kopfbereich ausgestattet, sodass neben einer optischen Inspektion eines entsprechenden Bereiches in geradliniger Richtung auch eine optische Inspektion des Bereiches in anderen Raumrichtungen erfolgen kann. Endoskope werden beispielsweise eingesetzt, um während medizinischer Eingriffe eine klare Sicht auf einen engen oder innerhalb eines Körpers befindlichen Bereich zu erlangen oder aber auch in technischen Bereichen, um technische Maschinen und deren schlecht zugängliche Bereiche zu inspizieren oder zu betrachten. Es sei darauf hingewiesen, dass die Begriffe "Endoskop", "endoskopisches System" oder auch "endoskopisches Instrument" häufig synonym genutzt werden.

Eine "Gelenkanordnung" kann jede mechanische Anordnung sein, welche dazu geeignet ist, in einer Achse oder mehreren Achsen verschwenkt zu werden. Beispielsweise ist eine solche Gelenkanordnung eine Abfolge von "Gelenkkörpern", welche jeweils aufeinander abwälzen und damit eine vielfältige Gelenkigkeit der Gelenkanordnung darstellen.

Ein "distaler Endabschnitt" kann jeder Bereich oder Abschnitt der Gelenkmechanik sein, welcher einer Aktuatorik oder einem Körper oder einer anderweitigen zentralen Baugruppe beabstandet und abgewandt ist.

Ein "Lenkelement" kann beispielsweise ein Seil, ein Band, ein Draht oder eine Litze sein. Ein solches Lenkelement kann dabei jedes mechanische Element sein, welches dazu geeignet ist, Zugkräfte und/oder Druckkräfte aufzunehmen. Auch kann ein solches Lenkelement nur teilweise Druckkräfte aufnehmen, sodass mittels des Lenkelementes hauptsächlich Zug, jedoch zusätzlich auch Druck ausgeübt werden kann. Insbesondere ist dann die Fähigkeit, Druck auszuüben geringer als die ermöglichte Aufnahme von Zugkräften.

Ein "proximaler Abschnitt" kann jeder Bereich oder Abschnitt der Gelenkmechanik sein, welcher einer Aktuatorik oder einem Körper oder einer anderweitigen zentralen Baugruppe nahe angeordnet oder zugewandt ist.

"Zugfest" fixiert beschreibt in diesem Zusammenhang ein Fixieren, bei welchem Zugkräfte übertragen werden können, also beispielsweise ein entsprechendes Lenkelement mit der Taumelscheibe so verbunden ist, dass Zugkräfte zwischen dem Lenkelement und der Taumelscheibe übertragen werden können. Beispielsweise ist dazu ein drahtförmiges Lenkelement so an der Taumelscheibe eingehängt, dass die Taumelscheibe mittels eines Zugs am Lenkelement bewegt werden kann. Entsprechend beschreibt "druckfest" eine Anordnung, bei welcher Druckkräfte übertragbar sind, also eine Anordnung, bei welcher mittels eines beispielsweise stabförmigen, in eine Biegerichtung elastischen Lenkelementes Druckkräfte auf eine Taumelscheibe übertragen werden können, um die Taumelscheibe zu bewegen. Die Begriffe "zugfest" und "druckfest" beschreiben hierbei eine Festigkeit im Rahmen einer üblichen, technisch sinnvollen Belastbarkeit in Abhängigkeit der verwendeten Werkstoffe und Geometrien und umfasst beispielsweise auch eine gewisse Elastizität, welche insbesondere durch E-Moduli der verwendeten Werkstoffe erzeugt sein kann.

Eine "Taumelscheibe" ist insbesondere ein ortsfestes, jedoch schwenkbewegliches, zumeist rundes Bauteil, welches gegenüber einer Hauptachse oder Längsachse, beispielsweise der Gelenkmechanik, neigbar ist. Eine solche Taumelscheibe kann dabei symmetrisch um ihren Umfang angeordnete Befestigungspunkte oder Verbindungspunkte für die Lenkelemente aufweisen.

Eine "Verschiebeeinrichtung" kann jede technische Einrichtung sein, welche geeignet ist, die Taumelscheibe entlang einer Achse, beispielsweise einer Längsachse der Gelenkanordnung, zu verschieben. Beispielsweise ist eine solche Verschiebeeinrichtung eine Hülse, eine teleskopische Einrichtung oder eine entsprechend aufeinander abgestimmte Anordnung von Wellen.

Eine "Montagespannung" ist die Spannung der Lenkelemente, welche für einen späteren Einsatz der Gelenkmechanik zweckmäßig ist und die zu erwartenden Belastungen auf die Lenkelemente ermöglicht, ohne eine Überlastung oder ein Durchhängen der Lenkelemente bei sachgerechtem Einsatz herbeizuführen.

Um das Arretieren der Verschiebeeinrichtung zuverlässig und insbesondere einfach durchführen zu können, erfolgt das Arretieren der Verschiebeeinrichtung mittels eines Schweißens, eines Lötens, eines Klebens, eines Klemmens und/oder eines Schraubens, sodass eine Schweißverbindung, eine Lötverbindung, eine Klebeverbindung, eine Klemmverbindung und/oder eine Schraubverbindung vorliegt.

Insbesondere kann damit mittels Schweißens und/oder Klebens eine dauerhafte, irreversibel befestigte Verbindung zum Arretieren der Verschiebeeinrichtung geschaffen werden. Somit kann die Gelenkmechanik während der Fertigung optimal eingestellt werden, sodass im späteren Anwendungsfall keine weitere Einstellung mehr vorgenommen werden kann.

Erfolgt das Arretieren der Verschiebeeinrichtung mittels Klemmens und/oder Schraubens, so kann ein reversibles Arretieren der Verschiebeeinrichtung erreicht werden. Damit ist insbesondere ermöglicht, die Verschiebeeinrichtung auch im späteren Anwendungsfall nachzujustieren oder im Rahmen einer Reparatur, einer Wartung oder eines Services im Sinne einer technischen Überprüfung für die Gelenkmechanik neu oder optimiert einzustellen.

In einer Ausführungsform erfolgt das Befestigen der Lenkelemente an der Taumelscheibe mittels Umformens, Schweißens, Lötens, Klebens, Klemmens und/oder Schraubens, sodass eine Formschlussverbindung, eine Schweißverbindung, eine Lötverbindung, eine Klebeverbindung, eine Klemmverbindung und/oder eine Schraubverbindung vorliegt.

Damit kann auch das Befestigen der Lenkelemente an der Taumelscheibe wahlweise irreversibel oder reversibel ausgeführt werden, sodass je nach Anwendungsfall und gewünschtem möglichen Eingriff nach der Herstellung der Gelenkmechanik ein Nachjustieren der Gelenkmechanik möglich ist oder nicht ermöglicht wird.

Um einen weiteren Ausgleich von Zugkräften, Druckkräften und/oder der Montagespannung auf unterschiedlichen Lenkelementen oder ein gleitendes Einstellen einer Zugkraft, Druckkraft und/oder der Montagespannung für ein Lenkelement zu ermöglichen, erfolgt das Befestigen der Lenkelemente an der Taumelscheibe mittels Umschlingens eines Halteelementes mit einem jeweiligen Lenkelement, sodass insbesondere ein Längenausgleich der Lenkelemente während des Spannens ermöglicht wird.

So kann beispielsweise eine Möglichkeit geschaffen werden, dass ein jeweiliges Lenkelement beim Spannen der Gelenkmechanik zunächst einen kleinen Weg frei an der Taumelscheibe gleiten kann und erst bei steigender Zugkraft bis hin zur Montagespannung in eine vollständig befestigte Lage übergeht. Beispielsweise ist dies mittels einer Keilverbindung oder einer selbsthemmenden Klemmverbindung ermöglicht. Weiterhin kann ein jeweiliges Lenkelement mit einem jeweiligen benachbarten Lenkelement an der Gelenkmechanik einstückig ausgebildet sein und um ein jeweiliges Halteelement umschlungen sein. Somit ist ein Längenausgleich zwischen benachbarten Lenkelementen während des Spannens ermöglicht.

Ein "Halteelement" ist dabei beispielsweise ein Haken, eine Welle, eine Rollenführung, eine Rolle oder ein anderes Element, an welchem ein Lenkelement temporär gehalten und dann befestigt werden kann. Insbesondere kann ein solches Halteelement auch ein Schlitz oder ein Doppelschlitz in der Taumelscheibe sein, durch welchen ein jeweiliges Lenkelement hindurchgeführt wird.

Ein "Längenausgleich" ist jede Möglichkeit, durch welche während des Spannens eine freie Länge der jeweiligen Lenkelemente zwischen Taumelscheibe und dem distalen Endabschnitt ermöglicht ist, insbesondere um Zugkräfte und/oder Druckkräfte am jeweiligen Lenkelement auszugleichen.

In einer weiteren Ausführungsform wird während des Spannens die Montagespannung gemessen und insbesondere das Arretieren der Verschiebeeinrichtung bei Erreichen eines Schwellwertes für die Montagespannung durchgeführt.

Durch ein solches Messen der Montagespannung kann sichergestellt werden, dass die Gelenkmechanik eine definierte und gewünschte Vorspannung erhält. Insbesondere erfolgt das Arretieren der Verschiebeeinrichtung dann bei Erreichen eines Schwellwertes für die Montagespannung, sodass das Gesamtsystem der Gelenkmechanik definiert vorgespannt ist. Weiterhin kann ein solches Messen zur Qualitätssicherung genutzt werden, indem der gemessene Wert für die Montagespannung in den Dokumenten für das jeweilige medizinische Instrument hinterlegt wird.

Ein "Schwellwert" kann dabei jeder Wert sein, welcher charakteristisch für eine wünschenswerte oder technisch notwendige Montagespannung ist. Beispielsweise ist ein solcher Schwellwert ein durch die Konstruktion der Gelenkmechanik vorgegebener Wert für eine maximal zulässige Montagespannung.

Um eine genaue Abwinkelung der Gelenkmechanik in der späteren Anwendung und eine entsprechende Selbstausrichtung des Systems zu gewährleisten, erfolgt während des Befestigens, des Spannens und/oder des Arretierens ein, insbesondere geradliniges, Ausrichten des distalen Endstücks und/oder der Taumelscheibe, sodass eine definierte, insbesondere geradlinige, Ausrichtung der Gelenkmechanik nach dem Montieren erreicht wird.

Mittels eines solchen Ausrichtens wird damit die Gelenkmechanik, insbesondere in einer geradlinigen Ausrichtung, montiert. Zudem kann die Gelenkmechanik damit so eingestellt sein, dass ein Selbstausrichten in Richtung dieser, insbesondere geradlinigen, Ausrichtung erfolgt, wenn keine Zugkräfte auf die Lenkelemente aufgebracht werden.

Ein "Ausrichten" kann dabei jedes mechanische Fixieren oder anderweitige Aufprägen einer gewünschten Ausrichtung der Gelenkmechanik sein. Beispielsweise kann dazu die Gelenkmechanik in eine Vorrichtung eingespannt und/oder eingelegt werden, sodass ein definiertes Ausrichten ermöglicht ist. Insbesondere ist eine solche Vorrichtung dann geradlinig ausgebildet, sodass mittels der Vorrichtung ein gradliniges Ausrichten der Gelenkmechanik ermöglicht ist.

In einem weiteren Aspekt wird die Aufgabe gelöst durch eine Gelenkmechanik für ein medizinisches Instrument, insbesondere für einen medizinischen Roboter oder für ein Endoskop, wobei die Gelenkmechanik eine stützende Gelenkanordnung mit einem Gelenkkörper oder mehreren Gelenkkörpern, mehrere mit einem distalen Endabschnitt verbundene, um die Gelenkanordnung angeordnete Lenkelemente zum Manipulieren des distalen Endabschnitts und eine an einem proximalen Abschnitt angeordnete Taumelscheibe zum mechanischen Ansteuern der Lenkelemente aufweist, wobei die Gelenkanordnung eine Verschiebeeinrichtung aufweist, und wobei die Taumelscheibe an der Verschiebeeinrichtung entlang einer Längsachse der Gelenkanordnung beweglich angeordnet ist, und die Verschiebeeinrichtung ein Arretiermittel zum Arretieren der entlang der Längsachse verschieblich angeordneten Taumelscheibe aufweist und die Taumelscheibe mittels des Arretiermittels arretiert ist, sodass eine Vorspannung und/oder Montagespannung der jeweiligen Lenkelemente fixiert ist..

Eine solche Verschiebeeinrichtung ermöglicht es auf einfache Weise, dass die Taumelscheibe mit den an der Taumelscheibe angebrachten Lenkelementen so verschoben werden kann, dass mittels der Taumelscheibe alle Lenkelemente gleichzeitig und insbesondere mit gleicher Spannung und/oder Montagespannung gespannt werden können.

Um die Gelenkmechanik möglichst kompakt ausführen zu können, ist die Taumelscheibe auf einer Kugelaufnahme der Verschiebeeinrichtung aufgenommen.

Eine "Kugelaufnahme" kann dabei jede kugelförmige oder mit einer kugelförmig ausgestalteten Oberfläche ausgeführte mechanische Einrichtung sein, auf welche die Taumelscheibe insbesondere gleichmäßig und möglichst widerstandsarm beweglich angeordnet und/oder aufgenommen ist.

Erfindungsgemäß weist die Verschiebeeinrichtung ein Arretiermittel zum Arretieren der entlang der Längsachse verschieblich angeordneten Taumelscheibe auf.

Mittels eines solchen Arretiermittels wird die verschieblich angeordnete Taumelscheibe dann sicher arretiert, sodass eine entsprechende Vorspannung und/oder Montagespannung der jeweiligen Lenkelemente fixiert wird.

Ein "Arretiermittel" kann dabei jedes mechanische, reversible oder irreversible, Mittel sein, welches ein Verschieben der Taumelscheibe entlang der Verschiebeeinrichtung sicher unterbindet.

Um das Arretiermittel irreversibel auszuführen, ist das Arretiermittel insbesondere als Schweißnaht, Klebenaht und/oder Quetschverbindung ausgebildet.

In einer Ausführungsform, in der das Arretiermittel reversibel ausgebildet ist, ist das Arretiermittel insbesondere als Klemmverbindung, Schraubverbindung und/oder Keilverbindung ausgebildet.

Um die Verschiebeeinrichtung derart auszuführen, dass die Verschiebeeinrichtung Bestandteil anderer Wirkkomponenten ist und damit die Gelenkmechanik besonders kompakt gebaut werden kann, weist die Verschiebeeinrichtung eine Teleskopwelle auf, wobei die Teleskopwelle insbesondere entlang der Längsachse angeordnet ist.

Eine solche Teleskopwelle kann dabei beispielsweise derart ausgeführt sein, dass die Verschiebeeinrichtung durch ein Teleskopieren der Teleskopwelle realisiert ist. Weiterhin kann die Teleskopwelle auch rotatorische Kräfte aufnehmen, sodass beispielsweise eine Aktorik an einem distalen Endbereich der Gelenkmechanik mittels der Teleskopwelle angetrieben werden kann. Weist die Teleskopwelle zudem einen Innenraum auf, ist also als Hohlwelle ausgebildet, so können zusätzliche Elemente, wie beispielsweise Lichtwellenleiter oder andere optische oder mechanische Instrumente innerhalb der Teleskopwelle geführt sein.

In einer Ausführungsform weist die Teleskopwelle einen entlang der Längsachse ortsfesten ersten Wellenabschnitt und einen entlang der Längsachse beweglichen zweiten Wellenabschnitt auf, wobei insbesondere der erste Wellenabschnitt am distalen Endbereich und der zweite Wellenabschnitt an der Taumelscheibe angeordnet ist.

Eine solche Teleskopwelle kann besonders vorteilhaft in die Gelenkmechanik integriert werden. Der ortsfeste Wellenabschnitt kann, wenn dieser am distalen Endbereich angeordnet ist, gleichzeitig als Fixpunkt für beispielsweise an der Gelenkmechanik angeordnete Endeffektoren genutzt werden. Der zweite Wellenabschnitt kann dann gemeinsam mit der Taumelscheibe zum Spannen der Lenkelemente in proximaler Richtung genutzt werden. Damit wird die Geometrie eines Anschlusses an einen Endeffektor durch das Spannen mittels des zweiten Wellenabschnittes nicht beeinflusst.

Ein "Wellenabschnitt" kann dabei jeder mechanische Teil einer Welle sein, welcher gemeinsam mit einem weiteren Wellenabschnitt die Teleskopwelle bildet. Weiterhin kann ein erster Wellenabschnitt und ein zweiter Wellenabschnitt auch einen Teil der Teleskopwelle bilden, beispielsweise ein Element einer nach dem Prinzip der Kardanwelle ausgeführten Gesamtwelle innerhalb der Gelenkmechanik.

Um eine solche Welle, welche beispielsweise als Antriebswelle innerhalb der Gelenkmechanik mehrteilig ausgeführt ist, besonders kompakt ausführen zu können, weist die Teleskopwelle einen Gelenkkörper auf.

In einer weiteren Ausführungsform weist die Teleskopwelle einen Kugelkopf zur beweglichen Aufnahme der Taumelscheibe auf.

Auf einem solchen Kugelkopf, insbesondere auf der Oberfläche eines solchen Kugelkopfes, kann die Taumelscheibe dann sehr kompakt und schwenkbeweglich aufgenommen werden, insbesondere in allen Richtungen oder um alle Achsen schwenkbeweglich. Damit kann die Gelenkmechanik noch kompakter ausgeführt sein.

Um die Lenkelemente sicher an der Taumelscheibe befestigen zu können, weist die Taumelscheibe Fixiermittel zum reversiblen oder irreversiblen Aufnehmen der Lenkelemente auf.

Mittels solcher Fixiermittel kann dann das jeweilige Lenkelement sicher an der Taumelscheibe befestigt werden, sodass die Montage der Gelenkmechanik sehr einfach erfolgen kann.

Ein "Fixiermittel" kann dabei jedes technische Mittel sein, welches geeignet ist, ein jeweiliges Lenkelement sicher und insbesondere zugfest an der Taumelscheibe zu befestigen.

In einer Ausführungsform ist das Fixiermittel eine Schweißverbindung, eine Klebeverbindung, eine Klemmverbindung, eine Schraubverbindung oder eine formschlüssige Verbindung.

In einem weiteren Aspekt wird die Aufgabe gelöst durch ein medizinisches Instrument, insbesondere durch einen medizinischen Roboter oder ein Endoskop oder einen Schaft eines Endoskopes, welcher oder welches eine Gelenkmechanik nach einer der beschriebenen Ausführungsformen aufweist.

Ein solches medizinisches Instrument, ein solcher medizinischer Roboter, ein solches Endoskop und/oder ein solcher Schaft ist oder sind gemäß der oben genannten Vorteile einfach zu montieren, besonders kompakt ausgeführt und kann oder können weiterhin einfach gewartet werden.

In den Zeichnungen ist ein Ausführungsbeispiel der Erfindung dargestellt. Die Zeichnungen, die Beschreibung und die Ansprüche enthalten zahlreiche Merkmale in Kombination. Der Fachmann wird die Merkmale zweckmäßigerweise auch einzeln betrachten und zu sinnvollen weiteren Kombinationen zusammenfassen.

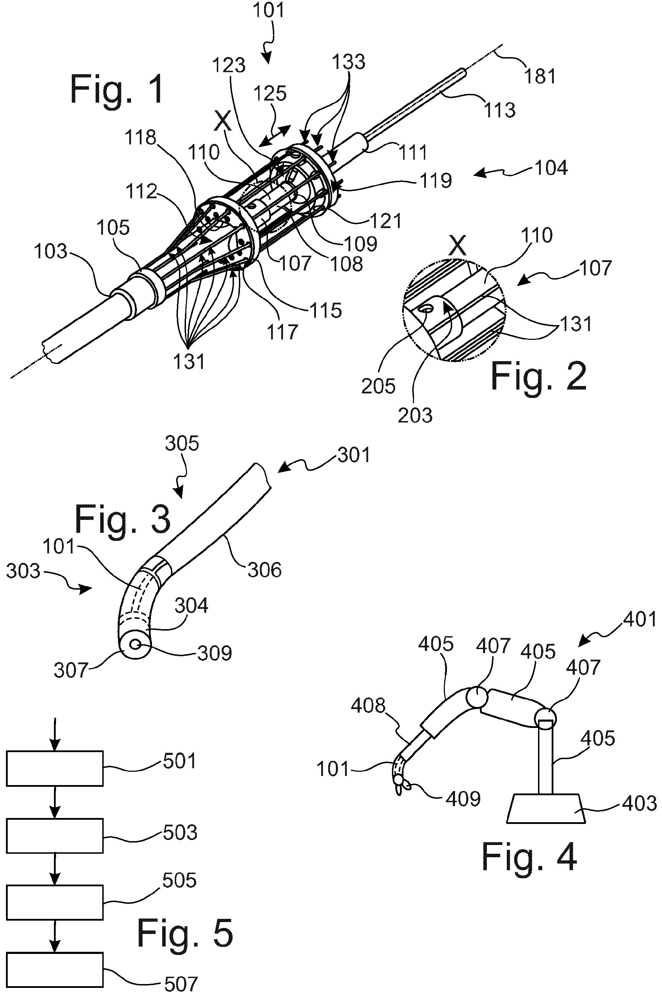

Im Weiteren wird die Erfindung anhand von Ausführungsbeispielen näher erläutert. Es zeigen
- Figur 1: eine schematische Darstellung einer Gelenkmechanik mit einer Verschiebeeinrichtung,
- Figur 2: eine vergrößerte Darstellung der Verschiebeeinrichtung der Figur 1,
- Figur 3: eine schematische Darstellung eines Endoskops mit einer Gelenkmechanik in isometrischer Ansicht,
- Figur 4: eine schematische Darstellung eines Roboters mit einem Endeffektor in einer Seitenansicht, sowie
- Figur 5: eine schematische Darstellung eines Verfahrensablaufes zum Montieren einer Gelenkmechanik.

Eine Gelenkmechanik 101 weist ein distales Endstück 103 auf. Am distalen Endstück 103 ist ein Haltering 105 angebracht. Innerhalb der Gelenkmechanik und innerhalb des distalen Endstücks 103 ist eine Hohlwelle 107 angeordnet. In der Hohlwelle 107 ist ein Schaft 110 einer Kugelwelle 108 längsverschieblich jedoch drehfest geführt. Die Kugelwelle 108 weist an einem proximalen Bereich 104 eine Kugelpfanne 109 auf, in welcher eine weitere Welle 111 schwenkbar jedoch drehfest aufgenommen ist. Die Kugelwelle 108 sowie die weitere Welle 111 sind ebenfalls als Hohlwelle ausgeführt. Die Hohlwelle 107, die Kugelwelle 108 sowie die Welle 111 bilden damit eine hohle, in Längsrichtung durchgängige Wellenverbindung nach dem Prinzip einer Kardanwelle.

Eine Hauptwelle 112 ist am distalen Endstück 103 und am Haltering 105 angeschlossen. Die Hauptwelle 112 umgibt die Hohlwelle 107. An der Hauptwelle 112 ist mittels eines Vierkants 118 ein Führungsring 115 aufgebracht. Der Führungsring 115 korrespondiert dabei an einem Innenbereich mit dem Vierkant 118, sodass der Führungsring 115 verdrehfest auf der Hauptwelle 112 aufgenommen ist.

Der Führungsring 115 weist Führungen 117 auf, durch welche Lenkdrähte 131 geführt sind. Die Lenkdrähte 131 sind dabei gleichmäßig rund um einen Umfang der Gelenkmechanik 101 angeordnet.

Auf einer Kugelfläche 123 der Kugelwelle 108 ist eine Taumelscheibe 119 schwenkbar angeordnet. Die Taumelscheibe gleitet dabei mit einer kugelförmigen Innenfläche (nicht dargestellt) auf der Kugelfläche 123. In der Taumelscheibe 119 sind die Lenkdrähte 131 in Anschlüssen 121 zugfest aufgenommen. In einem proximalen Bereich 104 sind die Lenkdrähte 131 mit einer jeweiligen Fügestelle 133 versehen, an welchen der Anschluss der Lenkdrähte 131 an weitere Ansteuerungsmechanik erfolgen kann.

Alle Komponenten sind symmetrisch um eine gemeinsame Achse 181 angeordnet. Innerhalb der Hohlwelle 107, der hohl ausgebildeten Kugelwelle 108 sowie der hohl ausgebildeten Welle 111 ist eine Antriebswelle 113 angeordnet, welche ebenfalls entlang der Achse 181 geführt ist. Die Antriebswelle 113 dient zum Weiterleiten mechanischer Drehmomente von einem proximalen Bereich 104 in das distale Endstück 103, sodass mittels der Antriebswelle 113 beispielsweise ein Endeffektor am distalen Endstück 103 der Gelenkmechanik 101 angetrieben werden kann.

Die Kugelwelle 108 ist innerhalb der Hohlwelle 107 längsverschieblich angeordnet. Dazu gleitet der Schaft 110 der Kugelwelle 108 in einer Muffe 203 an der Hohlwelle 107. An der Muffe 203 ist zudem ein radial angeordnetes Loch 205 angeordnet. Mittels dieser Anordnung aus Hohlwelle 107, Kugelwelle 108, Kugelfläche 123, Taumelscheibe 119 sowie der Muffe 203 kann die Gelenkmechanik 101 gespannt werden, dieser Vorgang wird im Folgenden detailliert erläutert:
Für ein Montieren der Gelenkmechanik wird zunächst der Zusammenbau des distalen Endstücks 103, des Halterings 105, des Führungsrings 115 sowie der Lenkdrähte 131 vorgenommen. Dabei werden die Lenkdrähte 131 am Führungsring 115 durch die Führungen 117 gefädelt und am Haltering 105 befestigt. Die Kugelwelle 108 wird dann vollständig in die Hohlwelle 107 eingeschoben, sodass die an der Kugelfläche 123 befestigte Taumelscheibe 119 ebenfalls weitestmöglich in Richtung des Führungsrings 115 bewegt ist. Sodann werden die Lenkdrähte 131 durch die Anschlüsse 121 in der Taumelscheibe 119 geführt und innerhalb der Anschlüsse 121 befestigt. Somit liegen die Lenkdrähte 131 im ungespannten oder nur schlaff gespannten Zustand zwischen Führungsring 115 und Taumelscheibe 119 vor.

Zum Spannen der Gelenkmechanik 101 wird nun die Kugelwelle 108 aus der Hohlwelle 107 entlang eines Weges 125 in Richtung des proximalen Bereichs 104 herausgezogen. Die auf der Kugelfläche 123 ortsfest an der Kugelwelle 108 angebrachte Taumelscheibe 119 bewegt sich damit ebenfalls in Richtung des proximalen Bereichs 104. Somit werden alle Lenkdrähte 131 gleichzeitig durch die Bewegung der Taumelscheibe 119 in Richtung des proximalen Bereichs 104 gespannt. Bei diesem Vorgang ist die Gelenkmechanik 101 in einer Vorrichtung eingelegt, sodass eine geradlinig Ausrichtung entlang der Achse 181 erfolgt (nicht gezeigt).

Zum Festsetzen der Gelenkmechanik 101 wird nun in das Loch 205 der Muffe 203 ein Schweißpunkt gesetzt, sodass die Kugelwelle 108 in Richtung der Achse 181 fest innerhalb der Hohlwelle 107 fixiert ist. Dazu verbindet der Schweißpunkt die Muffe 203 stoffschlüssig mit dem Schaft 110 der Kugelwelle 108. Somit ist mit wenig Aufwand ein gleichmäßiges und vollständiges Spannen und Montieren der Gelenkmechanik 101 erfolgt.

Ein Endoskop 301 weist an einem distalen Ende 303 einen Kopf 304 auf. In einem proximalen Bereich 305 weist das Endoskop 301 einen Schaft 306 auf. An dem Schaft 306 kann das Endoskop 301 geführt werden, beispielsweise mit einer Hand. Innerhalb des Kopfes 304 ist die Gelenkmechanik 101 beispielhaft angeordnet. Anstatt der Antriebswelle 113 ist hier jedoch innerhalb der Gelenkmechanik 101 ein optischer Leiter angeordnet, welcher in einem Objektiv 309 an einer Stirnfläche 307 des Kopfes 304 endet.

Das Endoskop 301 kann damit zur Beobachtung eines Arbeitsbereiches genutzt werden. Der Kopf 304 ist mittels der Gelenkmechanik 101 dabei frei um verschiedene Achsen schwenkbar. Dazu werden die Lenkdrähte 131 jeweils an der Seite mit Zugkraft belastet, in welche der Endoskopkopf 304 geschwenkt werden soll.

Ein Roboter 401 weist einen Fuß 403 sowie Arme 405 auf. Die Arme 405 sind mittels Gelenken 407 miteinander verbunden. An einem Arm 405, nämlich dem letzten Arm 405, ist ein Endeffektor 408 angebracht. Der Endeffektor 408 ist dabei ein feiner Greifer 409, wobei mittels des Greifers 409 medizinische Manipulationen vorgenommen werden können. Innerhalb des Greifers 409 ist dabei die Gelenkmechanik 101 mit der Antriebswelle 113 angeordnet. Somit kann der Greifer 409 mittels der Gelenkmechanik 101 frei verschwenkt werden. Mittels der Antriebswelle 113 wird ein Drehmoment zum Schließen und/oder Öffnen des Greifers 409 übertragen.

Der Ablauf des Verfahrens zum Montieren einer Gelenkmechanik, beispielsweise einer Gelenkmechanik 101, soll im Folgenden noch einmal erläutert sein:
Es erfolgt zunächst ein Vormontieren 501 des Gelenkmechanik 101 mit der Taumelscheibe 119, dem distalen Endabschnitt 103 sowie den Lenkdrähten 131. Danach erfolgt ein Befestigen 503 der Lenkdrähte 131 an der Taumelscheibe 119, sodass die Lenkdrähte 131 zwischen dem distalen Endabschnitt 103, nämlich dem Haltering 105, und der Taumelscheibe 119 zugfest fixiert sind. Sodann erfolgt ein Spannen 505 der Lenkdrähte 131 mittels eines axialen Verschiebens der Taumelscheibe 119, nämlich entlang eines Weges 125, sodass die Lenkdrähte 131 mit einer Spannung zwischen dem distalen Endbereich 103 und der Taumelscheibe 119 gespannt sind. Sodann erfolgt ein Festsetzen 507 der Taumelscheibe 119 an der Hohlwelle 107, sodass die Gelenkmechanik 101 montiert und mit gespannten Lenkdrähten 131 vorliegt.

In den Zeichnungen ist ein Ausführungsbeispiel der Erfindung dargestellt. Die Zeichnungen, die Beschreibung und die Ansprüche enthalten zahlreiche Merkmale in Kombination. Der Fachmann wird die Merkmale zweckmäßigerweise auch einzeln betrachten und zu sinnvollen weiteren Kombinationen zusammenfassen. Die Erfindung betrifft ein Verfahren zum Montieren einer Gelenkmechanik für ein medizinisches Instrument, insbesondere für einen medizinischen Roboter oder für ein Endoskop, wobei die Gelenkmechanik eine stützende Gelenkanordnung mit einem Gelenkkörper oder mehreren Gelenkkörpern, mehrere mit einem distalen Endabschnitt verbundene, um die Gelenkanordnung angeordnete Lenkelemente zum Manipulieren des distalen Endabschnitts und eine an einem proximalen Abschnitt angeordnete Taumelscheibe zum mechanischen Ansteuern der Lenkelemente aufweist. Des Weiteren betrifft die Erfindung eine Gelenkmechanik für ein medizinisches Instrument, insbesondere für einen medizinischen Roboter oder ein Endoskop, sowie ein medizinisches Instrument, insbesondere einen medizinischen Roboter oder ein Endoskop.

### Bezugszeichenliste

- 101: Gelenkmechanik
- 103: distales Endstück
- 104: proximaler Bereich
- 105: Haltering
- 107: Hohlwelle
- 108: Kugelwelle
- 109: Kugelpfanne
- 110: Schaft
- 111: Welle
- 112: Hauptwelle
- 113: Antriebswelle
- 115: Führungsring
- 117: Führung
- 118: Vierkant
- 119: Taumelscheibe
- 121: Anschluss
- 123: Kugelfläche
- 125: Weg
- 131: Lenkdraht
- 133: Fügestelle
- 181: Achse
- 203: Muffe
- 205: Loch
- 301: Endoskop
- 303: distales Ende
- 304: Kopf
- 305: proximaler Bereich
- 306: Schaft
- 307: Stirnfläche
- 309: Objektiv
- 401: Roboter
- 403: Fuß
- 405: Arm
- 407: Gelenk
- 408: Endeffektor
- 409: Greifer
- 501: Vormontieren
- 503: Befestigen
- 505: Spannen
- 507: Festsetzen

## Patentansprüche

1. Verfahren zum Montieren einer Gelenkmechanik (101) für ein medizinisches Instrument, insbesondere für einen medizinischen Roboter (401) oder für ein Endoskop (301), wobei die Gelenkmechanik eine stützende Gelenkanordnung (107, 108, 111) mit einem Gelenkkörper (107, 108, 111) oder mehreren Gelenkkörpern(107, 108, 111), mehrere mit einem distalen Endabschnitt (103) verbundene, um die Gelenkanordnung (107, 108, 111) angeordnete Lenkelemente (131) zum Manipulieren des distalen Endabschnitts (103) und eine an einem proximalen Abschnitt (104) angeordnete Taumelscheibe (119) zum mechanischen Ansteuern der Lenkelemente (131) aufweist, mit folgenden Schritten:
- Vormontieren der Gelenkanordnung (107, 108, 111) mit der Taumelscheibe (119) und dem distalen Endabschnitt (103) sowie den Lenkelementen (131), sodass eine Gelenkanordnung (107, 108, 111) mit dem distalen Endabschnitt (103), den Lenkelementen (131) und der Taumelscheibe (119) vorliegt und die Lenkelemente (131) zugfest mit dem distalen Endabschnitt (103) verbunden sind,
- Befestigen der Lenkelemente (131) an der Taumelscheibe (119), sodass die Lenkelemente (131) zwischen dem distalen Endabschnitt (103) und der Taumelscheibe (119) zugfest und/oder druckfest fixiert sind,
- Spannen der Lenkelemente (131) mittels eines axialen Verschiebens (125) der Taumelscheibe (119) mittels einer Verschiebeeinrichtung (110, 203) in proximaler Richtung (104) , sodass die Lenkelemente (131) mit einer Montagespannung zwischen dem distalen Endbereich (103) und der Taumelscheibe (119) gespannt sind, und
- Festsetzen der Taumelscheibe (119) an der Gelenkanordnung (107, 108, 111) in axialer Richtung mittels eines Arretierens der Verschiebeeinrichtung (110, 203), sodass die Gelenkmechanik montiert und mit gespannten Lenkelementen vorliegt.

2. Verfahren gemäß Anspruch 1, **dadurch gekennzeichnet, dass** das Arretieren der Verschiebeeinrichtung (110, 203) mittels eines Schweißens, eines Lötens, eines Klebens, eines Klemmens und/oder eines Schraubens erfolgt, sodass eine Schweißverbindung, eine Lötverbindung, eine Klebeverbindung, eine Klemmverbindung und/oder eine Schraubverbindung vorliegt.

3. Verfahren gemäß Anspruch 1 oder 2, **dadurch gekennzeichnet, dass** Befestigen der Lenkelemente (131) an der Taumelscheibe (119) mittels eines Umformens, eines Schweißens, eines Lötens, eines Klebens, eines Klemmens und/oder eines Schraubens erfolgt, sodass eine Formschlussverbindung, eine Schweißverbindung, eine Lötverbindung, eine Klebeverbindung, eine Klemmverbindung und/oder eine Schraubverbindung vorliegt.

4. Verfahren gemäß einem der vorherigen Ansprüche, **dadurch gekennzeichnet, dass** das Befestigen der Lenkelemente (131) an der Taumelscheibe (131) mittels eines Umschlingens eines Halteelementes mit dem jeweiligen Lenkelement (131) erfolgt, sodass insbesondere ein Längenausgleich der Lenkelemente (131) während des Spannens ermöglicht wird.

5. Verfahren gemäß einem der vorherigen Ansprüche, **dadurch gekennzeichnet, dass** während des Spannens die Montagespannung gemessen wird und insbesondere das Arretieren der Verschiebeeinrichtung (110, 203) bei Erreichen eines Schwellwertes für die Montagespannung erfolgt.

6. Verfahren gemäß einem der vorherigen Ansprüche, **dadurch gekennzeichnet, dass** während des Befestigens, des Spannens und/oder des Arretierens ein, insbesondere geradliniges, Ausrichten des distalen Endstückes (103) und/oder der Taumelscheibe (119) erfolgt, sodass eine definierte, insbesondere geradlinige, Ausrichtung der Gelenkmechanik nach dem Montieren erreicht wird.

7. Gelenkmechanik (101) für ein medizinisches Instrument, insbesondere für einen medizinischen Roboter (401) oder für ein Endoskop (301), wobei die Gelenkmechanik eine stützende Gelenkanordnung (107, 108, 111) mit einem Gelenkkörper (107, 108, 111) oder mehreren Gelenkkörpern (107, 108, 111), mehrere mit einem distalen Endabschnitt (103) verbundene, um die Gelenkanordnung (107, 108, 111) angeordnete Lenkelemente (131) zum Manipulieren des distalen Endabschnitts (103) und eine an einem proximalen Abschnitt (104) angeordnete Taumelscheibe (119) zum mechanischen Ansteuern der Lenkelemente (131) aufweist, und die Gelenkanordnung (107, 108, 111) eine Verschiebeeinrichtung (110, 203) aufweist, wobei die Taumelscheibe (119) an der Verschiebeeinrichtung (110, 203) entlang einer Längsachse (181) der Gelenkanordnung (107, 108, 111) beweglich angeordnet ist, wobei die Verschiebeeinrichtung (110, 203) ein Arretiermittel (203, 205) zum Arretieren der entlang der Längsachse verschieblich angeordneten Taumelscheibe (119) aufweist und die Taumelscheibe (119) mittels des Arretiermittels (203, 205) arretiert ist, sodass eine Vorspannung und/oder Montagespannung der jeweiligen Lenkelemente fixiert ist.

8. Gelenkmechanik gemäß Anspruch 7, **dadurch gekennzeichnet, dass** die Taumelscheibe (119) auf einer Kugelaufnahme (123) der Verschiebeeinrichtung (110, 203) aufgenommen ist.

9. Gelenkmechanik gemäß Anspruch 7 oder 8, **dadurch gekennzeichnet, dass** das Arretiermittel (203, 205) irreversibel, insbesondere als Schweißnaht, Klebenaht und/oder Quetschverbindung, ausgebildet ist.

10. Gelenkmechanik gemäß Anspruch 7 oder 8, **dadurch gekennzeichnet, dass** das Arretiermittel reversibel, insbesondere als Klemmverbindung, Schraubverbindung und/oder Keilverbindung, ausgebildet ist.

11. Gelenkmechanik gemäß einem der Ansprüche 7 bis 10, **dadurch gekennzeichnet, dass** die Verschiebeeinrichtung (110, 203) eine Teleskopwelle (107, 108) aufweist, wobei die Teleskopwelle (107, 108) insbesondere entlang der Längsachse angeordnet ist.

12. Gelenkmechanik gemäß Anspruch 11, **dadurch gekennzeichnet, dass** die Teleskopwelle (107, 108) einen entlang der Längsachse (181) ortsfesten ersten Wellenabschnitt (107) und einen entlang der Längsachse (181) beweglichen zweiten Wellenabschnitt (108) aufweist, wobei insbesondere der erste Wellenabschnitt (107) am distalen Endbereich (103) und der zweite Wellenabschnitt (108) an der Taumelscheibe (119) angeordnet ist.

13. Gelenkmechanik gemäß Anspruch 11 oder 12, **dadurch gekennzeichnet, dass** die Teleskopwelle (107, 108) einen Gelenkkörper (109) aufweist.

14. Gelenkmechanik gemäß einem der Ansprüche 11 bis 13, **dadurch gekennzeichnet, dass** die Teleskopwelle (107, 108) einen Kugelkopf (123) zur beweglichen Aufnahme der Taumelscheibe (119) aufweist.

15. Gelenkmechanik gemäß einem der Ansprüche 7 bis 14, **dadurch gekennzeichnet, dass** die Taumelscheibe (119) ein Fixiermittel (121) zum reversiblen oder irreversiblen Aufnehmen der Lenkelemente (131) aufweist.

16. Gelenkmechanik gemäß Anspruch 15, **dadurch gekennzeichnet, dass** das Fixiermittel eine Schweißverbindung, eine Klebeverbindung, eine Klemmverbindung, eine Schraubverbindung oder eine formschlüssige Verbindung ist.

17. Medizinisches Instrument, insbesondere medizinischer Roboter (401), Endoskop (301) oder Endoskopschaft (304, 306), mit einer Gelenkmechanik (101) gemäß einem der Ansprüche 7 bis 16.

## Claims

1. A method for assembling an articulated mechanism (101) for a medical instrument, in particular for a medical robot (401) or for an endoscope (301), wherein the articulated mechanism has a supporting articulated arrangement (107, 108, 111) with one articulated body (107, 108, 111) or a plurality of articulated bodies (107, 108, 111), a plurality of steering elements (131) connected to a distal end section (103) and arranged around the articulated arrangement (107, 108, 111) for manipulating the distal end section (103), and a swash plate (119) arranged on a proximal section (104) for mechanically activating the steering elements (131), having the following steps:
- pre-assembling the articulated arrangement (107, 108, 111) with the swash plate (119) and the distal end section (103), as well as the steering elements (131), such that an articulated arrangement (107, 108, 111) with the distal end section (103), the steering elements (131) and the swash plate (119) is present, and the steering elements (131) are connected to the distal end section (103) in a tensile-force-resistant manner,
- fastening the steering elements (131) to the swash plate (119) such that the steering elements (131) are fixed between the distal end section (103) and the swash plate (119) in a tensile-force-resistant and/or compressive-force-resistant manner,
**characterised by**
- tensioning the steering elements (131) by means of an axial displacement (125) of the swash plate (119) by means of a displacement device (110, 203) in the proximal direction (104), such that the steering elements (131) are tensioned with an assembly tension between the distal end region (103) and the swash plate (119),
- securing the swash plate (119) to the articulated arrangement (107, 108, 111) in the axial direction by means of locking the displacement device (110, 203),
such that the articulated mechanism is assembled and present with tensioned steering elements.

2. The method according to claim 1, **characterised in that** the displacement device (110, 203) is locked by means of welding, soldering, bonding, clamping and/or screwing, such that a welded connection, a soldered connection, a bonded connection, a clamped connection and/or a screwed connection is present.

3. The method according to claim 1 or 2, **characterised in that** the steering elements (131) are fastened to the swash plate (119) by means of forming, welding, soldering, bonding, clamping and/or screwing, such that a form-locking connection, a welded connection, a soldered connection, a bonded connection, a clamped connection and/or a screwed connection is present.

4. The method according to one of the preceding claims, **characterised in that** the steering elements (131) are fastened to the swash plate (131) by means of wrapping a retaining element around the respective steering element (131), such that, in particular, the length of the steering elements (131) can be compensated for during tensioning.

5. The method according to one of the preceding claims, **characterised in that** during tensioning, the assembly tension is measured and, in particular, the displacement device (110, 203) is locked when a threshold value for the assembly tension is reached.

6. The method according to one of the preceding claims, **characterised in that** during fastening, tensioning and/or locking, the distal end piece (103) and/or the swash plate (119) are aligned, in particular in a straight line, such that a defined, in particular straight-line, alignment of the articulated mechanism is achieved after assembly.

7. An articulated mechanism (101) for a medical instrument, in particular for a medical robot (401) or for an endoscope (301), wherein the articulated mechanism comprises a supporting articulated arrangement (107, 108, 111) with one articulated body (107, 108, 111) or a plurality of articulated bodies (107, 108, 111), a plurality of steering elements (131) connected to a distal end section (103) and arranged around the articulated arrangement (107, 108, 111) for manipulating the distal end section (103), and a swash plate (119) arranged on a proximal section (104) for mechanically activating the steering elements (131), and the articulated arrangement (107, 108, 111) has a displacement device (110, 203), wherein the swash plate (119) is arranged on the displacement device (110, 203) so as to be movable along a longitudinal axis (181) of the articulated arrangement (107, 108, 111), **characterised in that** the displacement device (110, 203) has a locking means (203, 205) for locking the swash plate (119), which is arranged so as to be displaceable along the longitudinal axis, and the swash plate (119) is locked by means of the locking means (203, 205) such that a pretension and/or assembly tension of the respective steering elements is fixed.

8. The articulated mechanism according to claim 7, **characterised in that** the swash plate (119) is received on a ball receptacle (123) of the displacement device (110, 203).

9. The articulated mechanism according to claim 7 or 8, **characterised in that** the locking means (203, 205) is designed to be irreversible, in particular as a weld seam, bond seam and/or crimped connection.

10. The articulated mechanism according to claim 7 or 8, **characterised in that** the locking means is designed to be reversible, in particular as a clamped connection, screwed connection and/or wedge connection.

11. The articulated mechanism according to one of claims 7 to 10, **characterised in that** the displacement device (110, 203) has a telescopic shaft (107, 108), wherein the telescopic shaft (107, 108) is arranged in particular along the longitudinal axis.

12. The articulated mechanism according to claim 11, **characterised in that** the telescopic shaft (107, 108) has a first shaft section (107) which is stationary along the longitudinal axis (181) and a second shaft section (108) which is movable along the longitudinal axis (181), wherein, in particular, the first shaft section (107) is arranged at the distal end region (103) and the second shaft section (108) is arranged on the swash plate (119).

13. The articulated mechanism according to claim 11 or 12, **characterised in that** the telescopic shaft (107, 108) has an articulated body (109).

14. The articulated mechanism according to one of claims 11 to 13, **characterised in that** the telescopic shaft (107, 108) has a ball head (123) for movably receiving the swash plate (119).

15. The articulated mechanism according to one of claims 7 to 14, **characterised in that** the swash plate (119) has a fixing means (121) for reversibly or irreversibly receiving the steering elements (131).

16. The articulated mechanism according to claim 15, **characterised in that** the fixing means is a welded connection, a bonded connection, a clamped connection, a screwed connection or a form-locking connection.

17. A medical instrument, in particular a medical robot (401), an endoscope (301) or an endoscope shaft (304, 306), having an articulated mechanism (101) according to one of claims 7 to 16.

## Revendications

1. Procédé d'assemblage d'un mécanisme d'articulation (101) pour un instrument médical, en particulier pour un robot médical (401) ou pour un endoscope (301), dans lequel le mécanisme d'articulation présente un agencement d'articulation de support (107, 108, 111) avec un corps d'articulation (107, 108, 111) ou plusieurs corps d'articulation (107, 108, 111), plusieurs éléments de guidage (131) reliés à une partie d'extrémité distale (103) et disposés autour de l'agencement d'articulation (107, 108, 111) pour manipuler la partie d'extrémité distale (103), et un plateau oscillant (119) disposé sur une partie proximale (104) pour commander mécaniquement les éléments de guidage (131), comprenant les étapes suivantes :
- le préassemblage de l'agencement d'articulation (107, 108, 111) avec le plateau oscillant (119) et la partie d'extrémité distale (103) ainsi que les éléments de guidage (131), de sorte qu'il existe un agencement d'articulation (107, 108, 111) avec la partie d'extrémité distale (103), les éléments de guidage (131) et le plateau oscillant (119) et que les éléments de guidage (131) soient reliés à la partie d'extrémité distale (103) de manière à résister à la traction,
- la fixation des éléments de guidage (131) sur le plateau oscillant (119), de sorte que les éléments de guidage (131) soient fixés entre la partie d'extrémité distale (103) et le plateau oscillant (119) de manière à résister à la traction et/ou à la compression,
**caractérisé par**
- la mise en tension des éléments de guidage (131) par un déplacement axial (125) du plateau oscillant (119) au moyen d'un dispositif de déplacement (110, 203) dans la direction proximale (104), de sorte que les éléments de guidage (131) soient mis en tension avec une tension de montage entre la zone d'extrémité distale (103) et le plateau oscillant (119), et
- l'immobilisation du plateau oscillant (119) sur l'agencement d'articulation (107, 108, 111) dans la direction axiale au moyen d'un verrouillage du dispositif de déplacement (110, 203),
de sorte que le mécanisme d'articulation soit monté et présente des éléments de guidage mis en tension.

2. Procédé selon la revendication 1, **caractérisé en ce que** le verrouillage du dispositif de déplacement (110, 203) s'effectue par soudage, brasage, collage, serrage et/ou vissage, de sorte qu'il existe une liaison soudée, une liaison brasée, une liaison collée, une liaison serrée et/ou une liaison vissée.

3. Procédé selon la revendication 1 ou 2, **caractérisé en ce que** la fixation des éléments de guidage (131) sur le plateau oscillant (119) s'effectue par formage, soudage, brasage, collage, serrage et/ou vissage, de sorte qu'il existe une liaison par complémentarité de formes, une liaison soudée, une liaison brasée, une liaison collée, une liaison serrée et/ou une liaison vissée.

4. Procédé selon l'une des revendications précédentes, **caractérisé en ce que** la fixation des éléments de guidage (131) sur le plateau oscillant (131) s'effectue au moyen d'un enroulement d'un élément de maintien autour de l'élément de guidage (131) respectif, de sorte qu'en particulier une compensation de longueur des éléments de guidage (131) soit rendue possible pendant la mise en tension.

5. Procédé selon l'une des revendications précédentes, **caractérisé en ce que**, pendant la mise en tension, la tension de montage est mesurée et, en particulier, le verrouillage du dispositif de déplacement (110, 203) a lieu lorsqu'une valeur seuil pour la tension de montage est atteinte.

6. Procédé selon l'une des revendications précédentes, **caractérisé en ce que**, pendant la fixation, la mise en tension et/ou le verrouillage, un alignement, en particulier rectiligne, de l'embout distal (103) et/ou du plateau oscillant (119) est effectué, de sorte qu'un alignement défini, en particulier rectiligne, du mécanisme d'articulation soit obtenu après l'assemblage.

7. Mécanisme d'articulation (101) pour un instrument médical, en particulier pour un robot médical (401) ou pour un endoscope (301), dans lequel le mécanisme d'articulation présente un agencement d'articulation de support (107, 108, 111) avec un corps d'articulation (107, 108, 111) ou plusieurs corps d'articulation (107, 108, 111), plusieurs éléments de guidage (131) reliés à une partie d'extrémité distale (103), disposés autour de l'agencement d'articulation (107, 108, 111) pour manipuler la partie d'extrémité distale (103), et un plateau oscillant (119) agencé sur une partie proximale (104) pour commander mécaniquement les éléments de guidage (131), et l'agencement d'articulation (107, 108, 111) présente un dispositif de déplacement (110, 203), dans lequel le plateau oscillant (119) est disposé de manière mobile sur le dispositif de déplacement (110, 203) le long d'un axe longitudinal (181) de l'agencement d'articulation (107, 108, 111), dans lequel le dispositif de déplacement (110, 203) présente un moyen de verrouillage (203, 205) pour verrouiller le plateau oscillant (119) disposé de manière coulissante le long de l'axe longitudinal et le plateau oscillant (119) est verrouillé par le moyen de verrouillage (203, 205), de sorte qu'une précontrainte et/ou une tension de montage des éléments de guidage respectifs soient fixées.

8. Mécanisme d'articulation selon la revendication 7, **caractérisé en ce que** le plateau oscillant (119) est reçu sur un logement sphérique (123) du dispositif de déplacement (110, 203).

9. Mécanisme d'articulation selon la revendication 7 ou 8, **caractérisé en ce que** le moyen de verrouillage (203, 205) est conçu de manière irréversible, en particulier sous la forme d'un cordon de soudure, d'un cordon de colle et/ou d'un sertissage.

10. Mécanisme d'articulation selon la revendication 7 ou 8, **caractérisé en ce que** le moyen de verrouillage est conçu de manière réversible, en particulier sous la forme d'une liaison par serrage, d'une liaison par vissage et/ou d'une clavetage.

11. Mécanisme d'articulation selon l'une des revendications 7 à 10, **caractérisé en ce que** le dispositif de déplacement (110, 203) présente un arbre télescopique (107, 108), dans lequel l'arbre télescopique (107, 108) est disposé en particulier le long de l'axe longitudinal.

12. Mécanisme d'articulation selon la revendication 11, **caractérisé en ce que** l'arbre télescopique (107, 108) présente une première partie d'arbre (107) fixe le long de l'axe longitudinal (181) et une seconde partie d'arbre (108) mobile le long de l'axe longitudinal (181), dans lequel, en particulier, la première partie d'arbre (107) est disposée au niveau de la partie d'extrémité distale (103) et la seconde partie d'arbre (108) est disposée au niveau du plateau oscillant (119).

13. Mécanisme d'articulation selon la revendication 11 ou 12, **caractérisé en ce que** l'arbre télescopique (107, 108) présente un corps d'articulation (109).

14. Mécanisme d'articulation selon l'une des revendications 11 à 13, **caractérisé en ce que** l'arbre télescopique (107, 108) présente une tête sphérique (123) pour le logement mobile du plateau oscillant (119).

15. Mécanisme d'articulation selon l'une des revendications 7 à 14, **caractérisé en ce que** le plateau oscillant (119) présente un moyen de fixation (121) pour recevoir de manière réversible ou irréversible les éléments de guidage (131).

16. Mécanisme d'articulation selon la revendication 15, **caractérisé en ce que** le moyen de fixation est une liaison soudée, une liaison collée, une liaison par serrage, une liaison vissée ou une liaison par complémentarité de formes.

17. Instrument médical, en particulier robot médical (401), endoscope (301) ou tige d'endoscope (304, 306), doté d'un mécanisme d'articulation (101) selon l'une des revendications 7 à 16.
